# EUROPEAN PATENT APPLICATION

(11) **EP 4 434 983 A1**
(43) Date of publication of application: **25.09.2024**
(21) Application number: 22894763.6
(22) Date of filing: 15.11.2022
(51) Int. Cl.: C07D 413/14, C07D 265/32, C07D 333/38

(54) **METHOD FOR SYNTHESIZING RIVAROXABAN**

(30) Priority: 17.11.2021 CN 202111361147
(71) Applicant: Zhejiang Huahai Pharmaceutical Co., Ltd., Taizhou, Zhejiang 317024 (CN); Zhejiang Huahai Licheng Pharmaceutical Co., Ltd., Taizhou, Zhejiang 317024 (CN)
(72) Inventor: HU, Jiaxing, Taizhou, Zhejiang 317024 (CN); ZHANG, Jian, Taizhou, Zhejiang 317024 (CN); ZHANG, Hao, Taizhou, Zhejiang 317024 (CN); ZHU, Zhenghe, Taizhou, Zhejiang 317024 (CN); WANG, Anyu, Taizhou, Zhejiang 317024 (CN); HUANG, Wenfeng, Taizhou, Zhejiang 317024 (CN); ZHU, Yan, Taizhou, Zhejiang 317024 (CN); JIN, Yongjun, Taizhou, Zhejiang 317024 (CN)
(74) Representative: Ostertag & Partner Patentanwälte mbB
(86) International application number: PCT/CN2022/131867
(87) International publication number: WO 2023/088229

(57) **Abstract**

Provided in the present invention is a method for synthesizing rivaroxaban. The method comprises: reacting an intermediate I with an intermediate II in an organic solvent in the presence of an alkali to obtain rivaroxaban, wherein the synthesis route is shown as follows, R in formula I is phenyl or substituted phenyl, and in substitution, the substituent is selected from nitro, halogen and C₁-C₆ alkyl; and X in formula II is bromine or chlorine. The method for synthesizing rivaroxaban provided in the present invention is short in terms of route, mild in terms of reaction conditions, simple in terms of post-treatment, high in terms of reaction yield and very suitable for industrial production

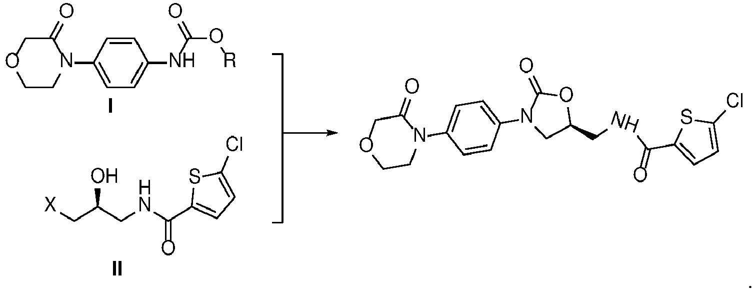

## Description

This application claims the priority to Chinese Patent Application No. 202111361147.X filed before the CNIPA on November 17, 2021, entitled "METHOD FOR SYNTHESIZING RIVAROXABAN", which is incorporated herein by reference in its entirety.

### TECHNICAL FIELD

The application relates to the field of organic synthesis, in particular to a method for synthesizing rivaroxaban.

### BACKGROUND

Rivaroxaban, chemically known as 5-chloro-N-[[(5S)-2-oxo-3-[4-(3-oxomorpholin-4-yl)phenyl]-1,3-oxazolidin-5-yl] methyl]thiophen-2-formamide, has a structural formula shown below:

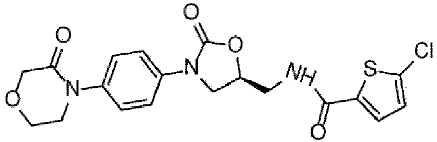

Rivaroxaban, a factor Xa inhibitor, is now one of the most commonly used anticoagulant medicaments in clinical practice since its introduction to the market due to its good efficacy and high safety.

WO2004060887A1 discloses a synthesis route of rivaroxaban, in which an intermediate represented by formula B and N-(4-aminophenyl)-3-morpholinone represented by formula C were used to synthesize a ring-opened rivaroxaban represented by formula D, and then the ring-opened rivaroxaban and N,N'-carbonyldiimidazole (CDI) constructed an oxazoline ring to obtain rivaroxaban, wherein, the yield of the ring-opened rivaroxaban represented by formula D in the route was only 61.8%, and thus the overall yield was not high. The synthesis route is shown as follows:

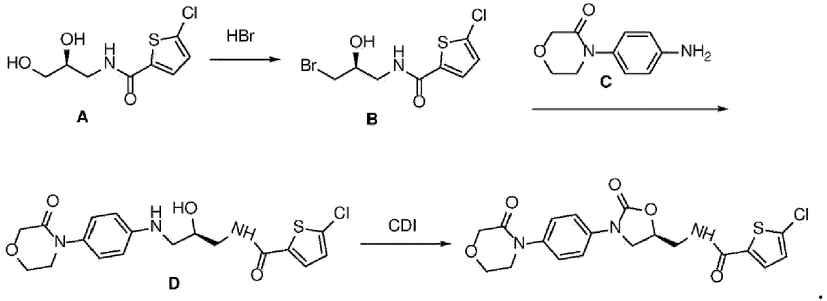

In CN102753537B, the intermediate represented by B in WO2004060887A1 was replaced with E, which was then reacted with intermediate C to obtain the ring-opened substance represented by formula D with a yield of 76%, and the synthesis route is shown as follows:

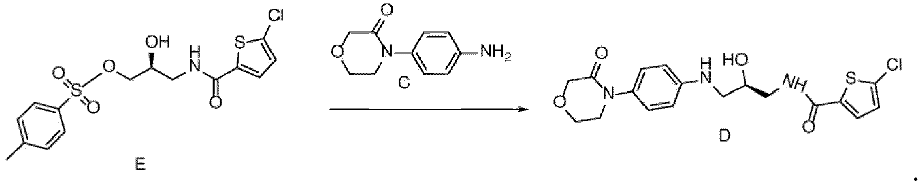

CN102753537B also discloses another synthesis route of rivaroxaban, in which E was acetylated and then cyclocondensed with F to obtain the compound rivaroxaban with a yield of only 21%, and the synthesis route is shown as follows:

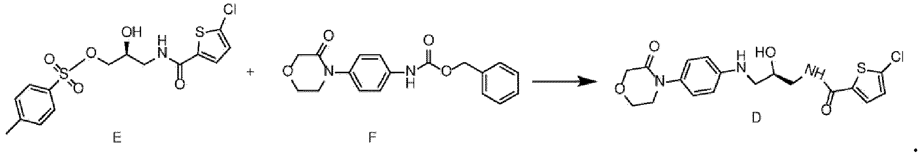

CN104693139B discloses reacting (S)-5-bromo-N-(3-chloro-2-hydroxypropyl)thiophen-2-formamide or (S)-5-chloro-N-(3-chloro-2-hydroxypropyl)thiophen-2-formamide with benzyl 4-(3-oxomorpholino))carbamate to obtain rivaroxaban with yields of 76% and 71%, respectively, which requires passing through a column after the completion of the reaction. The synthesis route is shown as follows:

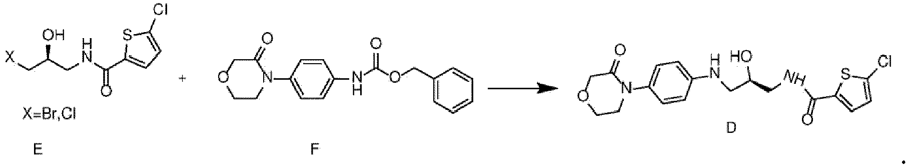

Therefore, there is a need to provide a novel method for synthesizing rivaroxaban.

### SUMMARY

An object of the application is to provide a method for synthesizing rivaroxaban to solve the problems of complex operation and low yield in the prior art.

The application provides a method for synthesizing rivaroxaban, comprising: reacting an intermediate I with an intermediate II in an organic solvent in the presence of an alkali to obtain rivaroxaban by a synthesis route shown as follows: 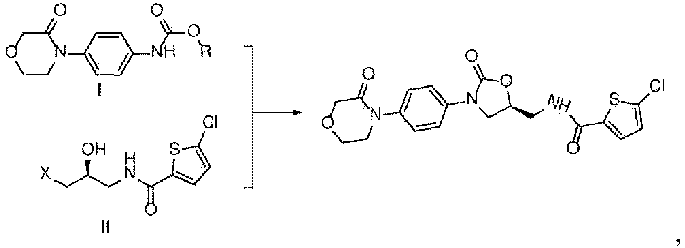
wherein, X in formula II is bromine or chlorine; and
R in formula I is phenyl or substituted phenyl, and in case of substituted phenyl, the substituent is selected from the group consisting of nitro, halogen and C₁-C₆ alkyl.

As a preferable embodiment of the application, the substituted phenyl is selected from the group consisting of p-nitrophenyl, 2,4-dinitrophenyl, 4-chlorophenyl and 4-methylphenyl.

As a preferable embodiment of the application, the alkali is selected from the group consisting of inorganic alkali and organic alkali. The inorganic alkali is selected from the group consisting of potassium hydroxide, sodium hydroxide, potassium carbonate, sodium carbonate, sodium hydride and calcium hydride; and the organic alkali is selected from the group consisting of lithium tert-butoxide, potassium tert-butoxide, sodium methoxide, potassium methoxide, sodium ethoxide, potassium ethoxide and pyridine. The inorganic alkali is further preferably sodium hydroxide or potassium hydroxide; and the organic alkali is further preferably lithium tert-butoxide, sodium methoxide or sodium ethoxide. The alkali is further preferably added in batches, for example, the alkali may be added to the reaction solution in 2 - 8 batches.

As a preferable embodiment of the application, the organic solvent is one solvent selected from the group consisting of ethers, haloalkanes, aromatic hydrocarbons, amides, esters, and C₃-C₆ ketones or any solvent mixture thereof, preferably one solvent selected from the group consisting of tetrahydrofuran, dichloromethane, 2-methyltetrahydrofuran, toluene, N,N-dimethylformamide, ethyl acetate, acetone, and methyl isobutyl ketone or any solvent mixture thereof.

In the application, the mass-to-volume ratio of the intermediate I to the organic solvent may be 1 g: 10 mL to 1 g: 50 mL.

As a preferable embodiment of the application, the molar ratio of intermediate I to intermediate II is 0.5: 1 to 2: 1, preferably 0.97: 1 to 1.5: 1, for example, the molar ratio of intermediate I to intermediate II may be 1: 1, 1.05: 1, 1.1: 1, 1.15: 1, 1.2: 1, 1.25: 1, 1.3: 1 or 1.4: 1.

As a preferable embodiment of the application, the molar ratio of the alkali to the intermediate I is 1: 1 to 4: 1, preferably 1 : 1 to 3: 1.

As a preferable embodiment of the application, the temperature of the reaction is -10°C to 50°C, preferably 0°C to 50°C.

As a preferable embodiment of the application, the duration of the reaction is 1 h to 12 h, preferably 2 h to 5 h.

As a preferable embodiment of the application, a post-treatment method of the reaction is as follows: after the completion of the reaction, adding acid to adjust the pH of the reaction solution to 7 to 8, concentrating under reduced pressure to remove the reaction solvent, adding a mixed solution of organic solvent and water , stirring the resultant slurry, and filtering to obtain rivaroxaban. The acid is preferably an aqueous hydrochloric acid solution. The organic solvent is preferably acetone or dichloromethane, more preferably acetone, most preferably a solvent mixture of acetone and water at a volume ratio of 4:3.

As a preferable embodiment of the application, intermediate I is synthesized by the following method:
reacting N-(4-aminophenyl)-3-morpholinone as starting material with chloroformate ROCOCl or di-tert-butyl dicarbonate (Boc₂O) to produce intermediate I by a synthesis route shown as follows: 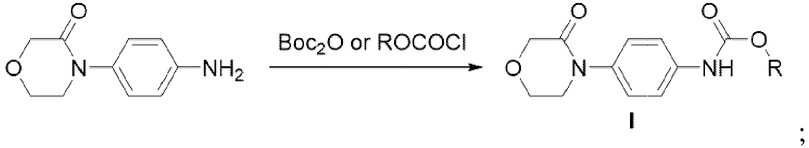
wherein, R in formula I is phenyl or substituted phenyl, and in case of substituted phenyl, the substituent is selected from the group consisting of nitro, halogen and C₁-C₆ alkyl.

In a preferable embodiment, for example, N-(4-aminophenyl)-3-morpholinone and chloroformate or di-tert-butyl dicarbonate are reacted with stirring at room temperature in a solvent mixture of dichloromethane and water in the presence of potassium bicarbonate to obtain Intermediate I.

As a preferable embodiment of the application, Intermediate II is synthesized by the following method:
reacting 5-chlorothiophen-2-formyl chloride with (S)-1-amino-3-chloro-2-propanol hydrochloride to produce Intermediate II by a synthesis route shown as follows:

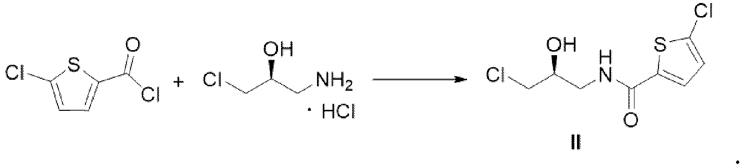

In a preferable embodiment, for example, a toluene solution of 5-chlorothiophen-2-formyl chloride is added to an aqueous sodium bicarbonate solution of (S)-1-amino-3-chloro-2-propanol hydrochloride, and the reaction is carried out with stirring at room temperature to obtain Intermediate II.

The method for synthesizing rivaroxaban provided by the present application has advantages of a short synthesis route, mild reaction conditions, simple post-treatment and high reaction yield, and thus is very suitable for industrial production.

### DETAILED DESCRIPTION

The application is further illustrated to make the objects, technical solutions and advantages of the embodiments more apparent. Obviously, the described examples are only a portion of the examples of the application and not all of them. On the basis of the examples in the application, all other examples obtained by those skilled in the art without inventive labor fall within the scope of protection of the application.

All the raw materials used in the examples are commercially available. The following specific examples are only preferable examples of the application for further detailed description of the application, and shall not be used to limit the scope of protection of the application.

### Example 1: Preparation of (S)-5-chloro-N-(3-chloro-2-hydroxypropyl)thiophen-2-formamide (Formula II)

120 g of (S)-1-amino-3-chloro-2-propanol hydrochloride, 138 g of sodium bicarbonate, and 600 mL of drinking water were added into a three-necked flask in turn. The mixture was stirred for 30 minutes, and then added with a toluene solution of 5-chlorothiophen-2-formyl chloride (163 g of 5-chlorothiophen-2-formyl chloride dissolved in 600 mL of toluene) slowly and dropwise at room temperature. After the completion of dropwise addition, a reaction was carried out for 2 h to 6 h at the same temperature. The resultant was filtered, and the filter cake was dried to obtain 198.3 g of (S)-5-chloro-N-(3-chloro-2-hydroxypropyl)thiophen-2-formamide as a white solid with a yield of 95% and a purity of 99.0%.

### Example 2: Preparation of N-(4-phenoxycarbonylaminophenyl)-3-morpholinone

34 g of potassium bicarbonate, 54 g of N-(4-aminophenyl)-3-morpholinone, 250 mL of dichloromethane, and 250 mL of drinking water were added into a three-necked flask in turn. The mixture was stirred at room temperature for 30 minutes, and then added with a dichloromethane solution of phenyl chloroformate (48.2 g of phenyl chloroformate dissolved in 350 mL of dichloromethane) slowly and dropwise for 1 h to 2 h. After the completion of dropwise addition, a reaction was carried out for 2 h to 6 h at the same temperature. The resultant was filtered, and the filter cake was dried to obtain 95.4 g of N-(4-phenoxycarbonylaminophenyl)-3-morpholinone as an off-white solid with a yield of 92% and a purity of 99.9%.

### Example 3: Preparation of N-(4-(p-nitrophenoxycarbonylaminophenyl)-3-morpholinone

34 g of potassium bicarbonate, 54 g of N-(4-aminophenyl)-3-morpholinone, 250 mL of dichloromethane, and 250 mL of drinking water were added into a three-necked flask in turn. The mixture was stirred at room temperature for 30 minutes, and then added with a dichloromethane solution of 4-nitrophenyl chloroformate (62.2 g of phenyl 4-nitrophenyl chloroformate dissolved in 350 mL of dichloromethane) slowly and dropwise for 1 h to 2 h. After the completion of dropwise addition, a reaction was carried out for 2 h to 6 h at the same temperature. The resultant was filtered, and the filter cake was dried to obtain 95.4 g of N-(4-(p-nitrophenoxycarbonylaminophenyl)-3-morpholinone as an off-white solid with a yield of 95% and a purity of 99.9%.

### Example 4: Preparation of Rivaroxaban

10 g of N-(4-phenoxycarbonylaminophenyl)-3-morpholinone, 8.9 g of (S)-5-chloro-N-(3-chloro-2-hydroxypropyl)thiophen-2-formamide, and 200 mL of tetrahydrofuran were added into a three-necked flask in turn. The mixture was cooled down to 0°C under stirring, added with 5.1 g of lithium tert-butoxide, and warmed up to room temperature. A reaction was carried out for 4 h at the same temperature, and the reaction process was monitored by TLC. After the completion of the reaction, 5% dilute hydrochloric acid solution was added dropwise to adjust the pH of the reaction mixture to 7 to 8, and tetrahydrofuran was removed by vacuum distillation. 80 mL of acetone and 60 mL of drinking water were added. The resultant slurry was stirred for 1 h at room temperature and then filtered, and the filter cake was dried to obtain 11.9 g of the target product rivaroxaban as a white solid with a yield of 85% and a purity of 99.5%.

### Example 5: Preparation of Rivaroxaban

10 g of N-(4-phenoxycarbonylaminophenyl)-3-morpholinone, 8.1 g of (S)-5-chloro-N-(3-chloro-2-hydroxypropyl)thiophen-2-formamide, and 200 mL of 2-methyltetrahydrofuran were added into a three-necked flask in turn. The mixture was cooled down to 0°C under stirring, added with 2.6 g of sodium hydroxide, and warmed up to 50°C. A reaction was carried out for 2 h at the same temperature, and the reaction process was monitored by TLC. After the completion of the reaction, 5% dilute hydrochloric acid solution was added dropwise to adjust the pH of the reaction mixture to 7 to 8, and 2-methyltetrahydrofuran was removed by vacuum distillation. 80 mL of acetone and 60 mL of drinking water were added. The resultant slurry was stirred for 1 h at room temperature and then filtered, and the filter cake was dried to obtain 11.2 g of the target product rivaroxaban as a white solid with a yield of 80% and a purity of 99.3%.

### Example 6: Preparation of Rivaroxaban

10 g of N-(4-phenoxycarbonylaminophenyl)-3-morpholinone, 16.3 g of (S)-5-chloro-N-(3-chloro-2-hydroxypropyl)thiophen-2-formamide, and 200 mL of dichloromethane were added into a three-necked flask in turn. The mixture was cooled down to -10°C under stirring and added with 5.5 g of potassium hydroxide, and the temperature was controlled at -10°C. A reaction was carried out for 6 h at the same temperature, and the reaction process was monitored by TLC. After the completion of the reaction, 5% dilute hydrochloric acid solution was added dropwise to adjust the pH of the reaction mixture to 7 to 8, and dichloromethane was removed by vacuum distillation. 80 mL of acetone and 60 mL of drinking water were added. The resultant slurry was stirred for 1 h at room temperature and then filtered, and the filter cake was dried to obtain 11.6 g of the target product rivaroxaban as a white solid with a yield of 83% and a purity of 99.6%.

### Example 7: Preparation of Rivaroxaban

10 g of N-(4-phenoxycarbonylaminophenyl)-3-morpholinone, 8.1 g of (S)-5-chloro-N-(3-chloro-2-hydroxypropyl)thiophen-2-formamide, and 200 mL of 2-methyltetrahydrofuran were added into a three-necked flask in turn. The mixture was cooled down to 0°C under stirring, added with 2.6 g of sodium hydroxide in 5 batches, and warmed up to room temperature. A reaction was carried out for 4 h at the same temperature, and the reaction process was monitored by TLC. After the completion of the reaction, 5% dilute hydrochloric acid solution was added dropwise to adjust the pH of the reaction mixture to 7 to 8, and 2-methyltetrahydrofuran was removed by vacuum distillation. 80 mL of acetone and 60 mL of drinking water were added. The resultant slurry was stirred for 1 h at room temperature and then filtered, and the filter cake was dried to obtain 12.6 g of the target product rivaroxaban as a white solid with a yield of 90% and a purity of 99.4%.

### Example 8: Preparation of Rivaroxaban

10 g of N-(4-(p-nitrophenoxycarbonyl)aminophenyl)-3-morpholinone, 8.7 g of (S)-5-chloro-N-(3-chloro-2-hydroxypropyl)thiophen-2-formamide, and 200 mL of toluene were added into a three-necked flask in turn. The mixture was cooled down to 0°C under stirring, added with 5.1 g of lithium tert-butoxide, and warmed up to room temperature. A reaction was carried out for 3 h at the same temperature, and the reaction process was monitored by TLC. After the completion of the reaction, 5% dilute hydrochloric acid solution was added dropwise to adjust the pH of the reaction mixture to 7 to 8, and toluene was removed by vacuum distillation. 80 mL of acetone and 60 mL of drinking water were added. The resultant slurry was stirred for 1 h at room temperature and then filtered, and the filter cake was dried to obtain 11.4g of the target product rivaroxaban as a white solid with a yield of 93% and a purity of 99.7%.

### Example 9: Preparation of Rivaroxaban

10 g of N-(4-(p-nitrophenoxycarbonyl)aminophenyl)-3-morpholinone, 10.2 g of (S)-5-chloro-N-(3-chloro-2-hydroxypropyl)thiophen-2-formamide, and 500 mL of ethyl acetate were added into a three-necked flask in turn. The mixture was cooled down to 0°C under stirring, added with 4.3 g of sodium methoxide, and warmed up to room temperature. A reaction was carried out for 12 h at the same temperature, and the reaction process was monitored by TLC. After the completion of the reaction, 5% dilute hydrochloric acid solution was added dropwise to adjust the pH of the reaction mixture to 7 to 8, and ethyl acetate was removed by vacuum distillation. 80 mL of acetone and 60 mL of drinking water were added. The resultant slurry was stirred for 1 h at room temperature and then filtered, and the filter cake was dried to obtain 10.9 g of the target product rivaroxaban as a white solid with a yield of 90% and a purity of 99.1%.

### Example 10: Preparation of Rivaroxaban

10 g of N-(4-(p-nitrophenoxycarbonyl)aminophenyl)-3-morpholinone, 10.6 g of (S)-5-chloro-N-(3-chloro-2-hydroxypropyl)thiophen-2-formamide, and 200 mL of acetone were added into a three-necked flask in turn. The mixture was cooled down to 0°C under stirring, added with 5.7 g of sodium ethoxide, and warmed up to 35°C. A reaction was carried out for 4 h at the same temperature, and the reaction process was monitored by TLC. After the completion of the reaction, 5% dilute hydrochloric acid solution was added dropwise to adjust the pH of the reaction mixture to 7 to 8, and acetone was removed by vacuum distillation. 80 mL of acetone and 60 mL of drinking water were added. The resultant slurry was stirred for 1 h at room temperature and then filtered, and the filter cake was dried to obtain 10.7 g of the target product rivaroxaban as a white solid with a yield of 88% and a purity of 99.8%.

### Example 11: Preparation of Rivaroxaban

10 g of N-(4-phenoxycarbonylaminophenyl)-3-morpholinone, 10.5 g of (S)-5-chloro-N-(3-bromo-2-hydroxypropyl)thiophen-2-formamide, and 200 mL of tetrahydrofuran were added into a three-necked flask in turn. The mixture was cooled down to 0°C under stirring, added with 5.1 g of lithium tert-butoxide, and warmed up to 26°C. A reaction was carried out for 2 h at the same temperature, and the reaction process was monitored by TLC. After the completion of the reaction, 5% dilute hydrochloric acid solution was added dropwise to adjust the pH of the reaction mixture to 7 to 8, and tetrahydrofuran was removed by vacuum distillation. 80 mL of acetone and 60 mL of drinking water were added. The resultant slurry was stirred for 1 h at room temperature and then filtered, and the filter cake was dried to obtain 12.7 g of the target product rivaroxaban as a white solid with a yield of 90% and a purity of 99.7%.

The foregoing are only preferred examples of the application and are not intended to limit the scope of protection of the application, and any modifications, equivalent substitutions, improvements and the like made within the spirit and principles of the application are included in the scope of protection of the application.

## Claims

1. A method for synthesizing rivaroxaban, comprising:
reacting an intermediate I with an intermediate II in an organic solvent in the presence of an alkali to obtain rivaroxaban, wherein a synthesis route is shown as follows: 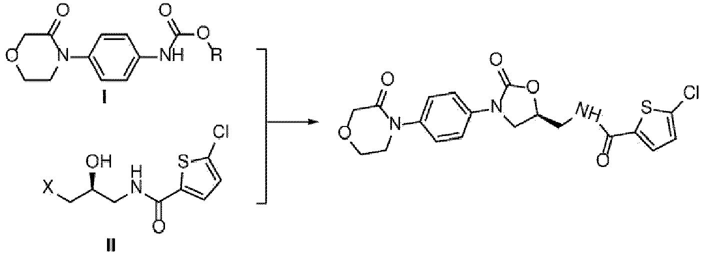
wherein, R in formula I is phenyl or substituted phenyl, and in case of the substituted phenyl, the substituent is selected from the group consisting of nitro, halogen and C₁-C₆ alkyl; and
X in formula II is bromine or chlorine.

2. The method of claim 1, wherein, the substituted phenyl is selected from the group consisting of p-nitrophenyl, 2,4-dinitrophenyl, 4-chlorophenyl and 4-methylphenyl.

3. The method of claim 1, wherein, the alkali is selected from the group consisting of inorganic alkali and organic alkali.

4. The method of claim 3, wherein, the inorganic alkali is selected from the group consisting of potassium hydroxide, sodium hydroxide, potassium carbonate, sodium carbonate, sodium hydride and calcium hydride, preferably sodium hydroxide or potassium hydroxide; and the organic alkali is selected from the group consisting of lithium tert-butoxide, potassium tert-butoxide, sodium methoxide, potassium methoxide, sodium ethoxide, potassium ethoxide and pyridine, preferably lithium tert-butoxide, sodium methoxide or sodium ethoxide.

5. The method of claim 1, wherein, the alkali is added in batches.

6. The method of claim 1, wherein, the organic solvent is one of ethers, haloalkanes, aromatic hydrocarbons, amides, esters and C₃-C₆ ketones, or any combination thereof, preferably one of tetrahydrofuran, 2-methyltetrahydrofuran, dichloromethane, toluene, N,N-dimethylformamide, ethyl acetate, acetone and methyl isobutyl ketone, or any combination thereof.

7. The method of claim 1, wherein, the molar ratio of the intermediate I to the intermediate II is 0.5: 1 to 2: 1, preferably 0.97: 1 to 1.5: 1.

8. The method of claim 1, wherein, the molar ratio of the alkali to the intermediate I is 1: 1 to 4: 1, preferably 1: 1 to 3: 1.

9. The method of claim 1, wherein, a temperature of the reaction is -10°C to 50°C, preferably 0°C to 50°C.

10. The method of claim 1, wherein, a duration of the reaction is 1 h to 12 h, preferably 2 h to 5 h.

11. The method of claim 1, wherein, a post-treatment method of the reaction is as follows: after completing the reaction, adding acid to adjust the pH of a reaction solution to 7 to 8, concentrating under reduced pressure to remove the solvent, adding a mixed solution of organic solvent and water, stirring the resultant slurry, and filtering to obtain rivaroxaban.

12. The method of claim 11, wherein, the acid is an aqueous hydrochloric acid solution.

13. The method of claim 11, wherein, the organic solvent is acetone or dichloromethane, preferably acetone.
